# EUROPÄISCHE PATENTSCHRIFT

(11) **EP 3 535 002 B1**
(45) Veröffentlichungstag und Bekanntmachung des Hinweises auf die Patenterteilung: **17.02.2021**
(21) Anmeldenummer: 17788235.4
(22) Anmeldetag: 25.10.2017
(51) Int. Cl.: A61M 16/04

(54) **TRACHEOSTOMIEKANÜLE**
TRACHEOSTOMY CANNULA
CANULE DE TRACHÉOTOMIE

(30) Priorität: 02.11.2016 DE 102016120821
(43) Veröffentlichungstag der Anmeldung: 11.09.2019
(73) Patentinhaber: Tracoe Medical GmbH, 55268 Nieder-Olm (DE)
(72) Erfinder: SCHNELL, Ralf, 63500 Seligenstadt (DE)
(74) Vertreter: WSL Patentanwälte Partnerschaft mbB
(86) Internationale Anmeldenummer: PCT/EP2017/077259
(87) Internationale Veröffentlichungsnummer: WO 2018/082980

(56) Entgegenhaltungen:
- GB-A- 2 324 735
- US-A1- 2008 283 052
- US-A1- 2010 288 289
- US-A1- 2013 000 649
- US-A1- 2014 000 627

## Beschreibung

Die vorliegende Erfindung betrifft eine Tracheostomiekanüle mit einem Tubus, welcher ein proximales Ende mit einer proximalen Öffnung und ein distales Ende mit einer distalen Öffnung und einen an seinem äußeren Umfang nahe des distalen Endes angeordneten Cuff aufweist.

Die erfindungsgemäße Tracheostomiekanüle ist insbesondere für eine Verwendung als Innenkanüle vorgesehen, die in eine bereits vorhandene Tracheostomiekanüle (Außenkanüle) ein- und hindurchführbar ist, so dass das distale Ende der Innenkanüle aus dem distalen Ende der Außenkanüle hervorsteht. Die Innenkanüle kann aber, insbesondere für kurze Übergangszeiten, auch allein ohne die Außenkanüle verwendet werden.

Darüber hinaus betrifft die vorliegende Erfindung auch eine Vorrichtung zur Atemluftzuführung, insbesondere Endotrachealtubus oder Tracheostomiekanüle, jeweils bestehend aus einer Innenkanüle und einer Außenkanüle mit einem Tubus, wobei der Tubus der Außenkanüle ein proximales Ende mit einer proximalen Öffnung und ein distales Ende mit einer distalen Öffnung aufweist.

Tracheostomiekanülen werden für die Zuführung von Atemluft durch einen künstlichen Zugang, das sog. Tracheostoma, in die Trachea eines Patienten eingeführt. Das proximale Ende des bogenförmig gekrümmten Tubus ist außerhalb des Körpers eines Patienten mit einer Beatmungsvorrichtung zur Atemluftzufuhr verbindbar, während das distale Ende der Tracheostomiekanüle innerhalb der Trachea des Patienten mündet. Der die zwei Enden miteinander verbindende Tubus dient der Hindurchleitung von Atemgas, sodass Atemgas über die Trachea in die Lunge des Patienten eingeleitet bzw. aus der Trachea zurückgeführt werden kann.

Für eine zuverlässige Versorgung mit Sauerstoff bzw. Atemgas ist es wichtig, dass der Tubus einer Tracheostomiekanüle, dessen Außendurchmesser zur Vermeidung von Verletzungen deutlich kleiner als der Innendurchmesser der Trachea ist, gegenüber der Innenwand der Trachea abgedichtet und möglichst kein oder wenig Atemgas an dem Tubus vorbei und über die oberen Atemwege zurückströmen kann bevor es in die Lunge gelangt. Hierfür ist an dem äußeren Umfang des Tubus nahe seines distalen Endes ein sogenannter Cuff (aufblasbare Manschette) angeordnet. Nach dem Einführen einer Tracheostomiekanüle in die Trachea eines Patienten wird der zunächst leere Cuff mit Gas (z. B. Luft) gefüllt und aufgebläht. Durch das Aufblähen legt sich der Cuff an die Innenwand der Trachea an und dichtet den Tubus gegenüber der Innenwand der Trachea ab. Der Cuff verhindert auch, dass Sekret aus dem subglottischen Raum an dem Tubus vorbei in die Bronchien gelangt. Solches Sekret enthält zumeist Bakterien, die bei einer Aspiration zu Pneumonien führen können, die eine häufige Komplikation bei langzeitbeatmeten Patienten darstellen.

Da die Tracheostomiekanüle in der Regel für eine Langzeitbeatmung vorgesehen ist, soll sie den Patienten möglichst wenig beeinträchtigen, die Schleimhaut der Trachea nicht beschädigen und eine sichere Beatmung des Patienten ermöglichen.

Aus der US 2013/000649 A1 ist bereits eine Tracheostomiekanüle mit den Merkmalen des Oberbegriffs des Anspruchs 1 bekannt.

Auch die US 2008/0283052 A1, die US 2014/0000627 A1, die US 2010/0288289 A1 zeigen bereits Trascheostomiekanülen mit einem im Bereich eines Cuffs verjüngten, ringförmigen Abschnitt auf der Außenseite eines Kanülenrohres angeordnet sind.

Stellt sich nach dem Einsetzen heraus, dass die Tracheostomiekanüle für den zu behandelnden Patienten zu groß, zu klein, zu kurz und/oder zu lang ist, kann sie Schmerzen verursachen oder es kann sogar ihre Funktion beeinträchtigt sein. Die Tracheostomiekanüle muss dann durch eine besser passende Tracheostomiekanüle ersetzt werden. Darüber hinaus können Situationen auftreten, in denen die Tracheostomiekanüle beschädigt oder verunreinigt ist oder aus anderen Gründen ausgewechselt werden muss. Bei der Entfernung einer herkömmlichen Tracheostomiekanüle aus der Trachea ist es praktisch unvermeidbar, dass Sekret, welches sich zwischenzeitlich angesammelt hat, an dem Tubus vorbei in die Bronchien und in die Lunge gelangt.

Demgegenüber besteht eine Aufgabe der vorliegenden Erfindung darin, eine Tracheostomiekanüle bereitzustellen, die einen sicheren Kanülenwechsel ermöglicht und dabei die Gefahr einer Aspiration von Sekret deutlich verringert.

Erfindungsgemäß wird diese Aufgabe durch eine Tracheostomiekanüle mit den Merkmalen des Anpruchs 1 gelöst. Bei dieser Tracheostomiekanüle weist der Tubus nahe seines distalen Endes einen sich zumindest über die axiale Länge des Cuffs erstreckenden verjüngten Abschnitt mit reduziertem Außendurchmesser auf, der einen kleineren Außendurchmesser hat als der Tubus in dem proximal davorliegenden Hauptabschnitt, wobei vorzugsweise die Länge des verjüngten Abschnittes mindestens der Länge des Cuffs entspricht und weniger als ein Drittel der Länge des Tubus beträgt und wobei weiterhin bevorzugt das proximale Ende des Cuffs um mindestens zwei Drittel der Tubuslänge von dem proximalen Ende des Tubus beabstandet ist Weiterhin wiest der Ringabschnitt mit dem kleineren Außendurchmesser D_{TR} eine Absaugöffnung auf, die proximal von dem Cuff an dem äußeren Umfang des Tubus (der Innenkanüle) angeordnet ist. Die proximal von dem Cuff angeordnete Absaugöffnung dient dem Absaugen von sich möglicherweise proximal vor dem Cuff ansammelnden Sekret. Dies ist insbesondere beim Wechsel von Kanülen von Bedeutung weil dann der Cuff bewegt und gegebenenfalls entleert wird und die Abdichtung nicht durchgehend sicher gewährleisten kann.

Die erfindungsgemäße Tracheostomiekanüle ist dafür vorgesehen, in eine zweite Tracheostomiekanüle eingebracht zu werden. Im Sinne der vorliegenden Erfindung wird die zweite Tracheostomiekanüle auch als Außenkanüle und die erste, innenliegende Tracheostomiekanüle als Innenkanüle bezeichnet. Die Tracheostomiekanüle ist jedoch auch ohne weiteres, insbesondere kurzzeitig, allein verwendbar, wobei die Vorteile der erfindungsgemäßen Tracheostomiekanüle vor allem in Verbindung mit einer Außenkanüle realisiert werden.

Der Abschnitt mir reduziertem Durchmesser kann z. B. stufenförmig von dem proximal davor liegenden Abschnitt des Tubus abgesetzt sein oder auch als eine mindestens teilweise konische Verjüngung der Außenseite des Tubus im distalen Bereich ausgestaltet sein. Sinn der Verjüngung ist die Aufnahme des eng anliegenden Cuffs im ungefüllten Zustand in der Weise, dass die Kanüle als Innenkanüle zusammen mit dem im verjüngten Abschnitt außen anliegenden Cuff durch eine Außenkanüle hindurchgeführt werden kann, ohne den Cuff zu beschädigen, wobei der Außendurchmesser im proximalen Bereich des Tubus dem Innendurchmesser der Außenkanüle entsprechen kann. Die Innenkanüle einschließlich des entleerten und außen im verjüngten Bereich anliegenden Cuff hat also in dem verjüngten Abschnitt einen Durchmesser, der nicht über den Durchmesser des Hauptabschnitts im proximalen Bereich hinausgeht.

Die Innenkanüle weist z. B. einen Tubus auf, der ebenso wie bei der Außenkanüle bogenförmig gekrümmt ist. Die Innenkanüle kann aber auch als gerade Kanüle ausgebildet sein, die erst beim Einführen durch die Außenkanüle deren gebogene Form annimmt. Der Tubus hat ein proximales Ende mit einer proximalen Öffnung sowie ein distales Ende mit einer distalen Öffnung. Dabei bezeichnet das "distale Ende" der jeweiligen Kanüle im Sinne der vorliegenden Erfindung das in die Trachea eingeführte bzw. in die Trachea einführbare Ende, das von einem behandelnden Arzt oder medizinischem Personal weiter entfernt liegt als das proximale Ende. Die Begriffe "distal" (fern gelegen) und "proximal" (nahe gelegen) werden also aus der Sicht einer behandelnden Person definiert. Entsprechend ist das "proximale Ende" für die Anordnung außerhalb des Körpers eines Patienten vorgesehen.

In einem Zustand, in dem die Innenkanüle in eine Außenkanüle eingebracht ist, ist der an dem äußeren Umfang nahe des distalen Endes des Tubus der Innenkanüle angeordnete Cuff jenseits des distalen Endes des Tubus der Außenkanüle angeordnet, um gegenüber der Innenwand der Trachea abdichten zu können. Im Sinne der vorliegenden Erfindung ist der Cuff "nahe des distalen Endes des Tubus" angeordnet, wenn der Cuff innerhalb eines axialen Endabschnittes des Tubus angeordnet ist, der, gemessen von dem distalen Ende des Tubus, sich über einen axialen Abschnitt des Cuffs erstreckt, dessen Länge maximal das Dreifache und minimal das Einfache des Innendurchmessers des Tubus beträgt.

In einer Ausführungsform beträgt der Abstand zwischen dem freien distalen Ende des Tubus und dem distalen Ansatzpunkt des Cuffs zwischen 1 mm und 10 mm, vorzugsweise zwischen 3 mm und 6 mm. Der Abstand zwischen dem distalen Ansatzpunkt des Cuffs und dem freien distalen Ende des Tubus soll verhindern, dass sich der Cuff bei einem Zurückziehen der Innenkanüle aus dem Tubus einer Außenkanüle vor die distale Öffnung des Tubus der Innenkanüle legt. Der proximale Ansatzpunkt des Cuffs kann beispielsweise in einem Abstand von 15 mm bis 40 mm von dem distalen Ende des Tubus liegen. Der Durchmesser des Cuffs, welcher senkrecht zur Tubus-Achse gemessen wird, beträgt minimal das Doppelte des Tubus-Innendurchmessers und maximal das Vierfache. Um zu verhindern, dass sich der Cuff der Innenkanüle versehentlich vor die distale Öffnung des Tubus der Innenkanüle legt wird eine Cuff-Form bevorzugt, bei der sich der Cuff in Richtung des distalen Endes konisch verjüngt.

Damit die Innenkanüle in die Außenkanüle einbringbar ist, muss notwendigerweise der Innendurchmesser des Tubus der Außenkanüle etwas größer als der Außendurchmesser des Tubus der Innenkanüle sein. Gemäß einer Ausführungsform sind der Innendurchmesser des Tubus der Außenkanüle und der Außendurchmesser des Tubus der Innenkanüle derart aufeinander abgestimmt, dass die Innenkanüle mit geringem Widerstand in der Außenkanüle gleiten kann.

Damit der Tubus der Innenkanüle samt dem an seinem äußeren Umfang angeordneten Cuff in den Tubus einer Außenkanüle einführbar ist, ist erfindungsgemäß vorgesehen, dass der Tubus an seinem distalen Ende einen sich zumindest über die axiale Länge des Cuffs erstreckenden Abschnitt aufweist dessen Außendurchmesser D_{TR} kleiner als der Außendurchmesser des Tubus D_{TPC} in dem proximal vor dem Ringabschnitt liegenden Abschnitt ist, der nachstehend als Hauptabschnittbezeichnet wird, so dass der leere an dem verjüngten Abschnitt anliegende Cuff den Gesamtdurchmesser nicht oder nicht wesentlich über den Außendurchmesser des Tubus außerhalb des verjüngten Abschnitts vergrößert. Der verjüngte Abschnitt wird nachstehend auch als Ringabschnitt bezeichnet, da er zumindest in bevorzugten Varianten eine Rotationsymmetrie aufweist.

Demnach steht der äußere Umfang des Cuffs in einem leeren und an den äußeren Umfang des Tubus angelegten Zustand nicht über den äußeren Umfang des Tubus in dem proximal vor dem Cuff liegenden Hauptabschnitt über. In seinem leeren und an den äußeren Umfang des Tubus angelegten Zustand, kann der Cuff vollständig in einem durch den kleineren Außendurchmesser D_{TR} gebildeten Ringraum aufgenommen werden. Beim Einführen des Tubus der Innenkanüle in den Tubus einer Außenkanüle ist der Cuff auf dieser Weise in dem Ringraum angeordnet und vor Beschädigungen beim Ein- bzw. Hindurchführen geschützt. Die verwendeten Cuffs haben bevorzugt eine geringe Wandstärke zwischen 5 µm und 45 µm. Im leeren und an den äußeren Umfang des Tubus angelegten Zustand weist der Cuff im Allgemeinen noch Falten auf, die radial nach außen abstehen können, Diese muss man gegebenenfalls mit geringer Kraft (z. B. 0,03 N) an den Tubus andrücken. Wenn diese Falten an den Tubus bzw. an den entleerten Cuff angelegt sind, passt der Tubus mit dem Cuff problemlos in die entsprechende Außenkanüle. Insbesondere wenn der Cuff beispielsweise eine Niederdruckmanschette ("High Volume Low Pressure Cuff") ist, können nach dem Entleeren des Cuffs zunächst schmale Falten des Cuffmaterials über den äußeren Umfang des Tubus in dem proximal vor dem Cuff liegenden Hauptabschnitt überstehen. Beim Einführen der Innenkanüle in eine Außenkanüle, werden diese Falten problemlos an den Tubus bzw. den entleerten Cuff angedrückt, sodass das Einführen nicht erschwert und der Cuff auch nicht beschädigt wird.

Der kleinere Außendurchmesser D_{TR} des verjüngten Abschnittes ist über die axiale Länge des verjüngten Abschnittes nicht notwendigerweise konstant, insbesondere wenn der verjüngte Abschnitt z. B. durch eine konische Verjüngung gebildet wird. Auch eine Kreisform oder Ringform des durch die Verjüngung gebildeten Freiraums ist nicht zwingend notwendig. Wesentlich ist lediglich, das durch die Verjüngung ein Freiraum zwischen der Außenseite des Tubus im verjüngten Bereich und einer gedachten Fortsetzung seines Hauptabschnittes vorliegt, die gleichzeitig auch eine radial innere Grenze für den Verlauf der Innenseite einer Außenkanüle definiert. Dieser Freiraum ermöglicht und erleichtert somit das Einbringen der Innenkanüle mit dem Cuff in und durch die Außenkanüle, ohne dass dabei der in dem Ringabschnitt angeordnete Cuff durch starke Reibung beschädigt wird. Der Tubus besteht im Wesentlichen aus einem Hauptabschnitt mit einem proximalen und einem distalen Ende, wobei der Ringabschnitt mit reduzierten Außendurchmesser nahe des distalen Endes liegt und das proximale Ende gegebenenfalls einen zusätzlich vergrößerten Außendurchmesser haben kann. Der Hauptabschnitt macht den größten Teil der Länge des Tubus aus und erstrecht sich proximal von dem Ringabschnitt. Mit "Durchmesser des Tubus" ist im Allgemeinen der Außendurchmesser des Hauptabschnitts gemeint. Der Innendurchmesser des Tubus ist vorzugsweise über seine gesamte Länge hinweg konstant. Es versteht sich, dass man den Grundgedanken eines verjüngten Abschnittes gegenüber dem Hauptabschnitt auch auf einen konischen Tubus, d. h. eine Tubus mit leicht konischem Hauptabschnitt anwenden kann, indem die Verjüngung eine zusätzliche Verjüngung gegenüber der gedachten Fortsetzung des konischen Hauptabschnittes darstellt.

Muss eine in die Trachea eines zu behandelnden Patienten eingeführte Tracheostomiekanüle herausgenommen oder ausgewechselt werden, kann zunächst die erfindungsgemäße Innenkanüle in die vorhandene Tracheostomiekanüle eingebracht werden, die dann als Außenkanüle dient. Die in der Trachea angeordnete Außenkanüle dient dabei als Führung für die Innenkanüle. Währenddessen oder anschließend kann die Innenkanüle die Funktion der Außenkanüle übernehmen, indem ihre proximale Öffnung beispielsweise über einen aufsteckbaren 15 mm Konnektor mit einer Beatmungseinrichtung verbunden wird. Der Cuff der Innenkanüle wird nach dem Hindurchführen durch die Außenkanüle aufgebläht und dichtet dann die Innenkanüle in der Trachea nach außen ab. Dann wird der Cuff der Außenkanüle entleert und die Außenkanüle kann von der Innenkanüle abgezogen und aus der Trachea und dem Tracheostoma entfernt werden.

Der Cuff der Innenkanüle verhindert, dass sich bei und nach dem Entfernen der Außenkanüle Sekret, welches sich an oder oberhalb des Cuffs der Außenkanüle in dem subglottischen Raum angesammelt hat, in die Bronchien gelangt.

Da die erfindungsgemäße Innenkanüle mindestens zeitweise allein für die Zufuhr der Atemluft zu einem Patienten verwendet wird, ist es zweckmäßig wenn sie an ihrem proximalen Ende mit einem 15 mm Standardanschluss versehen ist, der auch lösbar mit dem proximalen Ende des Hauptabschnitts verbunden sein kann.

Die Innenkanüle kann darüber hinaus (ohne den 15 mm Konnektor) auch als Führung für eine neu über die Innenkanüle in die Trachea einzuführende Außenkanüle verwendet werden.

Sekret, welches beim Wechsel der Außenkanüle oder zwischen der Außenkanüle und der Innenkanüle hinabfließt, wird durch den Cuff der Innenkanüle aufgehalten und kann durch die proximal von dem Cuff angeordnete Absaugöffnung abgesaugt werden. Nach dem Aufschieben einer neuen Außenkanüle auf die Innenkanüle, kann der Cuff der Außenkanüle aufgebläht werden und dichtet nunmehr die Außenkanüle in der Trachea ab. Anschließend kann gegebenenfalls nochmals eine Absaugung von Sekret aus dem Raum oberhalb des Cuffs der Innenkanüle (d. h. zwischen dem Cuff der Innenkanüle und dem Cuff der Außenkanüle) erfolgen, und die Innenkanüle kann dann nach dem Entleeren ihres Cuffs wieder aus der Außenkanüle zurückgezogen werden. Auf diese Weise kann ein sicherer Wechsel einer Tracheoestomiekanüle erfolgen, ohne dass die Gefahr besteht, dass Sekret während des Wechsels in die Bronchien oder die Lunge eines Patienten gerät, weil ununterbrochen eine Abdichtung entweder durch den Cuff der Außenkanüle oder durch den Cuff der Innenkanüle gewährleistet ist. Die vorhandene bzw. für die längere Behandlungsdauer vorgesehene Tracheostomiekanüle hat dabei die Funktion der Außenkanüle und die erfindungsgemäße Tracheostomiekanüle übernimmt die Funktion der Innenkanüle.

In einer Ausführungsform weist der Ringabschnitt im Bereich der Absaugöffnung einen stufenförmigen Übergang zwischen dem Außendurchmesser D_{TR} und D_{TPC} auf. Die Stufe zwischen den Außendurchmessern D_{TR} und D_{TPC} trennt den Ringabschnitt in dem der Cuff angeordnet ist und den Hauptabschnitt. Die Absaugöffnung ist in einer Ausführungsform im Bereich dieser Stufe und proximal vor dem Cuffansatz angeordnet. Die Stufe kann auch die Form eines konischen Übergangs haben.

Zum Absaugen von Sekret ist die Absaugöffnung mit einer Absaugleitung verbunden, wobei die Absaugleitung in einer Ausführungsform mit dem Tubus verklebt oder in die Wand des Tubus integriert ist. Eine mit dem Tubus verklebte oder in die Wand des Tubus integrierte Absaugleitung ist vor Beschädigungen beim Ein- oder Ausführen der Innenkanüle in eine Außenkanüle geschützt. Eine verklebte oder in das Material eingelassene Absaugleitung birgt auch nicht die Gefahr, dass die Absaugleitung beim Ein- oder Ausführen der Innenkanüle in einer Außenkanüle eingeklemmt wird. An ihrem proximalen Ende ist die Absaugleitung mit einer Saug- und/oder Spülvorrichtung verbindbar. Das proximale Ende der Absaugleitung kann an dem proximalen Ende des Tubus der Innenkanüle enden oder als Schlauchfortsatz ausgeführt sein.

In einer Ausführungsform weist der Cuff der Innenkanüle in seinem gefüllten Zustand eine maximale axiale Ausdehnung von 5 mm bis 30 mm, vorzugsweise 10 mm bis 20 mm, auf.

In einer Ausführungsform ist der Cuff der Innenkanüle eine entfaltbare Niederdruckmanschette, die mit einem Fluid befüllbar ist.

In einer weiteren Ausführungsform weist der Cuff einen proximalen Abschnitt auf, dessen Außendurchmesser sich in einem gefüllten Zustand des Cuffs nach proximal verjüngt, und/oder einen distalen Abschnitt, dessen Außendurchmesser sich in einem gefüllten Zustand des Cuffs nach distal verjüngt, auf. Die sich verjüngenden Abschnitte tragen dazu bei, dass sich der Cuff in seinem entleerten Zustand nicht über seinen proximalen und/oder distalen Ansatzpunkt hinaus erstreckt. Insbesondere verhindert der sich nach distal verjüngende Abschnitt, dass sich der leere Cuff vor die distale Öffnung des Tubus der Innenkanüle legen kann. Der sich nach proximal verjüngende Abschnitt und/oder der sich nach distal verjüngende Abschnitt verhindert außerdem, dass Teile des Cuffs beim Ein- und/oder Ausführen der Innenkanüle in eine bzw. aus einer Außenkanüle zwischen den Kanülen eingeklemmt werden.

Um möglicherweise an der Innenseite der Trachea oder an der Außenseite der Innenkanüle oder der Außenseite einer Außenkanüle herabfließendes Sekret zu sammeln, weist der Cuff in einer Ausführungsform einen proximal angeordneten Abschnitt mit einer Tellerform auf. Der tellerförmige, sich vorzugsweise relativ zu der Längsachse der Innenkanüle rotationssymmetrische erstreckende Abschnitt, erstreckt sich quer zu der Längsachse der Innenkanüle und bildet so einen Abschnitt zum Auffangen von anfallendem Sekret.

In einer Ausführungsform hat der Tubus an seinem proximalen Ende einen Außendurchmesser D_{TP}, der größer als der Außendurchmesser des Tubus D_{TPC} des Hauptabschnittes ist, wobei der Außendurchmesser D_{TP} um mindestens 3 % vorzugsweise um maximal 20 %, z. B. um etwa 10 %, größer ist als der Außendurchmesser D_{TPC}. Der proximale Abschnitt mit dem größeren Außendurchmesser D_{TP} verhindert, dass sich die Innenkanüle ungewollt weiter in den Tubus einer Außenkanüle hineinbewegt, wobei vorausgesetzt ist, dass der Innendurchmesser der Außenkanüle um weniger als 5 % vom Außendurchmesser des Hauptabschnittes abweicht. Der Abschnitt mit dem größeren Außendurchmesser D_{TP} kann auch als Mittel zum Befestigen der Innenkanüle in einer Außenkanüle verwendet werden, insbesondere in Verbindung mit einem konischen Übergang zum Hauptabschnitt. Der Abschnitt mit dem größeren Außendurchmesser D_{TP} kann dabei nach Art einer Presspassung in den Tubus einer Außenkanüle gedrückt und so befestigt werden.

In einer weiteren Ausführungsform weist das proximale Ende des Tubus kein Kanülenschild auf. Ein Kanülenschild dient im Allgemeinen der Befestigung einer Tracheostomiekanüle bei einer Dauerbehandlung und verhindert, dass die in die Trachea eingeführte Kanüle verrutscht. Weil aber die Innenkanüle im Allgemeinen nur für eine kurzzeitige Verwendung vorgesehen oder aber an einer Außenkanüle befestigbar ist bzw. durch ein Anschlussstück an einem vollständigen Hineinrutschen in die Außenkanüle gehindert ist, es ist es nicht erforderlich, dass die Innenkanüle ein Kanülenschild aufweist. Die Innenkanüle kann an der Außenkanüle befestigt werden.

Zum Anschluss an eine Vorrichtung zur Atemluftzuführung weist das proximale Ende des Tubus in einer Ausführungsform einen 15 mm Konnektor auf.

In einer Ausführungsform weist der Tubus ein Phonationsfenster auf, welches proximal zu dem Cuff an dem distalen Ende des Tubus angeordnet ist. Zweckmäßigerweise besteht das Phonationsfenster aus einer oder mehreren Durchbrechungen in der Wand des Tubus. Vorzugsweise ist das Phonationsfenster proximal zu einer Absaugöffnung angeordnet. Wird in einer Vorrichtung zur Atemluftzuführung eine Innenkanüle und eine Außenkanüle verwendet, so ist das Phonationsfenster der Innenkanüle zweckmäßigerweise in einem Bereich des Tubus der Innenkanüle angeordnet, der aus der distalen Öffnung der Außenkanüle hervorsteht. Alternativ kann die Außenkanüle ebenfalls ein Phonationsfenster proximal zum Cuff der Innenkanüle aufweisen, welches zweckmäßigerweise in eine mit dem Phonationsfenster der Innenkanüle mindestens teilweise überlappende Position gebracht werden kann.

In einer Ausführungsform weist die Trachealkanüle ein axial luftdurchlässiges Gelenk auf, wobei das Gelenk das proximale Ende und das distale Ende des Tubus (Kanülenrohrs) miteinander verbindet und mindestens eine begrenzte Beweglichkeit und insbesondere Verschwenkbarkeit der Achse des distalen Endes bezüglich der Achse des proximalen Endes ermöglicht. Das proximale Ende des Tubus wird auch als proximaler Rohrabschnitt und das distale Ende als distaler Rohrabschnitt bezeichnet.

Durch das Gelenk sind das proximale Ende und das distale Ende des Tubus relativ zueinander bewegbar, d. h. die Achsen der beiden Enden des Tubus sind verschwenkbar und unter unterschiedlichen relativen Winkeln einstellbar. Als Achse werden hier die zentralen Längsmittellinien der rohrförmigen Tubenabschnitte bezeichnet, die auch bogenförmig gekrümmt sein können und die ohne Gelenk zwischen dem proximalen und dem distalen Ende glatt und ohne Knick gegebenenfalls in einer leichten Krümmung ineinander übergehen würden, wobei das Gelenk eine relative Verschwenkung bzw. einen leichten Knick das Achsenverlaufes mit einem vergleichsweise kleine Krümmungsradius ermöglichen soll, der weniger als die Hälfte, vorzugsweise weniger als ein Fünftel des sonstigen minimalen Krümmungsradius des Tubus beträgt. Das distale Ende des Tubus kann somit gegenüber dem proximalen Ende des Tubus in verschiedene Richtungen verschwenkt werden. Während des Einführens der Kanüle in die Trachea und auch danach kann sich das proximale Ende des Tubus zusammen mit dem Kehlkopf und der Trachea bewegen, ohne dass das distale Ende des Tubus gegen die Wand der Trachea drückt oder an dieser reibt. Die gelenkige Verbindung des proximalen Endes mit dem distalen Ende erhöht die Beweglichkeit der Trachealkanüle und erleichtert das Schlucken.

In einer Ausführungsform weist der Tubus auf seinem äußeren Umfang einen befüllbaren Cuff zur Abdichtung und weichen Fixierung des Tubus in der Trachea ist das Gelenk zweckmäßigerweise in einem Bereich angeordnet, der durch die äußeren distalen und proximalen Befestigungsränder des Cuffs begrenzt ist.

Bei einer solchen Ausführungsform besteht ein Cuff aus einem aufblasbaren, sehr dünnwandigen Schlauch, dessen Endabschnitte außen auf dem Tubus eng anliegen und dort befestigt sind und der mindestens in einem zentralen Abschnitt gegenüber dem Tubus ein deutliches Übermaß hat oder auf ein solches Übermaß aufblasbar ist, so dass dieser Abschnitt sich nach dem Einführen der Kanüle in die Trachea aufblasen lässt und mit der Wand der Trachea entlang seines Umfangs abdichtend und mit vorzugsweise geringem Druck in Kontakt kommt. Die Endabschnitte, die im Allgemeinen ihrerseits Schlauchabschnitte (hier auch "Halsabschnitte" genannt) darstellen, sind typischerweise über ihre Länge und ihren Umfang hinweg mit der Außenwand des Kanülenrohres (Tubus) dicht verklebt oder verschweißt. Die axial äußeren Ränder dieser Endabschnitte legen dann die Grenzen des vorstehend definierten Bereiches für eine bevorzugte Anordnung eines Gelenks fest.

In einer Ausführungsform sind das proximale Ende und das distale Ende des jeweiligen Tubus durch einen ringförmigen Spalt mit einer lichten Spaltbreite d voneinander beabstandet und durch das axial luftdurchlässige Gelenk miteinander verbunden, welches den Spalt überbrückt.

In einer Ausführungsform befindet sich der ringförmige Spalt in einem axialen Bereich eines distalen oder proximalen Halsabschnittes eines Cuffs. D. h. der End- bzw. Halsabschnitt des Cuffs verbindet das proximale und distale Ende des jeweiligen Tubus miteinander und wirkt entweder selbst als Gelenk oder umfasst ein entsprechendes Gelenk. Die Wandstärke und Elastizität des Halsabschnittes muss gegebenenfalls an diesen Zweck angepasst sein.

Vorzugsweise ist das Gelenk mit dem proximalen Ende und/oder dem distalen Ende verklebt, verschweißt und/oder angespritzt. Damit durch die Gelenkverbindung kein Atemgas entweichen kann, ist in einer Ausführungsform vorgesehen, dass das Gelenk gasdicht mit dem proximalen Ende und dem distalen Ende des Tubus verbunden ist. Vorzugsweise ist das Gelenk radial luftundurchlässig.

In einer Ausführungsform weist das distale Ende relativ zu der Achse des proximalen Endes über das Gelenk einen maximalen Biegewinkel in einem Bereich von mindestens +10° bis -10°, vorzugsweise einen Bereich von +25° bis -25°, in beliebiger Richtung relativ zur Tubusachse auf.

Zweckmäßig ist das Gelenk derart ausgestaltet, dass der Gelenkwinkel zwischen dem proximalen Ende und dem distalen Ende mit einer um mehr als 70 % verringerten Kraft F um 10° veränderbar ist, als sie aufgewendet werden muss, um das proximale Ende ohne Gelenk um 10° zu verbiegen. Ein derart ausgestaltetes Gelenk ist ausreichend flexibel um einen Schluckprozess nicht zu erschweren und dennoch ausreichend fest, um ein einfaches Einführen der Trachealbeatmungsvorrichtung in eine Trachea zu ermöglichen.

In einer Ausführungsform sind sowohl der Tubus, d. h. dessen proximales und distales Ende, als auch das Gelenk aus Kunststoff hergestellt, wobei das Gelenk aus einem Kunststoff hergestellt ist, der im Vergleich zu dem Kunststoff des Tubus (Kanülenrohrs) eine geringere Shore-Härte aufweist. Der Kunststoff des Gelenks mit der im Vergleich zu dem Kunststoff des Kanülenrohrs geringeren Shore-Härte ist weicher und daher leichter verformbar und ermöglicht auf diese Weise eine Gelenkbewegung zwischen dem proximalen und distalen Rohrabschnitt. Die im Vergleich zu dem Kunststoff des Gelenks aus einem härteren Kunststoff hergestellten Rohrabschnitte sind hinreichend fest, um einfach in die Trachea eines Patienten eingeführt zu werden. Die aus einem härteren Kunststoff hergestellten Rohrabschnitte haben vorzugsweise eine Shore-A-Härte von 50 bis 98. Der im Vergleich hierzu weichere Kunststoff des Gelenks hat eine um mindestens 50 % geringere Shore-A-Härte.

Die entsprechende Gelenkbeweglichkeit lässt sich auch mit dem gleichen Kunststoffmaterial erzielen, wie es für die proximalen und distalen Rohrabschnitte verwendet wird, das jedoch in dem vorgesehenen Gelenkbereich eine geringere Wandstärke aufweist Die Wandstärke der proximalen und distalen Rohrabschnitte beträgt vorzugweise zwischen 0,8 mm und 1,8 mm. Demgegenüber beträgt die Wandstärke des Gelenks vorzugsweise zwischen 0,1 mm bis 0,4 mm. Weist das Gelenk einen Schlauchabschnitt auf, so kann die Wandstärke des Schlauchabschnittes deutlich verringert werden. Beispielsweise kann die Wandstärke eines Schlauchabschnittes 10 µm betragen.

In einer Ausführungsform ist das Gelenk in axialer Richtung des Kanülenrohrs elastisch dehnbar und/oder stauchbar, wobei die axiale Spaltbreite d des ringförmigen Spalts zwischen dem proximalen Rohrabschnitt und dem distalen Rohrabschnitt die maximale Stauchbarkeit vorgibt. Die Spaltbreite d ist in einer Ausführungsform 1 mm bis 10 mm, vorzugsweise 2 mm bis 4 mm. Die Stauchbarkeit ist so eingestellt, dass der distale Rohrabschnitt in Richtung des proximalen Rohrabschnittes bewegt wird, wenn der distale Rohrabschnitt mit einer Kraft größer als 0,05 N auf die Trachealwand drückt. Die elastische Stauchbarkeit und Dehnbarkeit sind durch die Verwendung eines entsprechend dünnen und/oder weichen Gelenkmaterials einstellbar.

Wenn eine solches dehn- und/oder stauchbares Gelenk innerhalb eines zentralen Cuff angeordnet ist, dämpft der Cuff die Relativbewegung zwischen dem proximalen Rohrabschnitt und dem distalen Rohrabschnitt. Werden der proximale Rohrabschnitt und der distale Rohrabschnitt aufeinander zubewegt, sinkt das Cuffvolumen und der Cuffdruck steigt. Vergrößert sich der Abstand zwischen dem proximalen Rohrabschnitt und dem distalen Rohrabschnitt dehnt sich das Volumen des Cuffs aus und der Cuffdruck sinkt. Umgekehrt kann man über eine Einstellung des Cuffdrucks innerhalb gewisser Grenzen auch die Dehnung bzw. das Maß des elastischen Zusammenziehens dehnbarer elastischer Schlauchabschnitte des Gelenks einstellen.

Der Cuffdruck ermöglicht eine Pufferung des Stauchens der Kanüle in axialer Richtung des Kanülenrohrs. Das Gelenk und der das Gelenk umgebende, unter leichtem Überdruck mit Gas befüllte Cuff wirken wie ein Stoßdämpfer, der Stöße auf die Trachealbeatmungsvorrichtung aufnimmt und verringert. Auf diese Weise können Schädigungen der Trachealwand durch das distale Kanülenende reduziert und der Schluckvorgang erleichtert werden. Vorzugsweise wird ein Cuff mit Hilfe von Luft mit einem Druck von 15 mbar bis 30 mbar beaufschlagt. Der radial außenliegend zu dem ringförmigen Spalt angeordnete Cuff bewirkt eine Rückstellkraft in die Ausgangslage des Gelenks. Zugleich verhindert der befüllte Cuff, dass die an dem Spalt befindlichen Enden des proximalen und des distalen Rohrabschnittes gegen die Trachea drücken und diese schädigen. Der Cuff polstert den ringförmigen Spalt und das Gelenk der Trachealbeatmungsvorrichtung gegenüber der Trachea ab, so dass eine bündige Fixierung eines das Gelenk bildenden Schlauchabschnittes an den jeweiligen Rohrabschnitten nicht erforderlich ist.

In einer Ausführungsform ist das Gelenk ein Schlauchabschnitt, der auf der einen Seite eines ringförmigen Spalts zwischen dem proximalen und dem distalen Rohrabschnitt an dem proximalen Rohrabschnitt und auf der anderen Seite des Spalts an dem distalen Rohrabschnitt befestigt ist. Vorzugsweise weist der Kunststoff des Schlauchabschnitts eine im Vergleich zu dem Kunststoff des Kanülenrohrs geringere Shore-Härte auf. Der Schlauchabschnitt aus dem weicheren Kunststoff ist elastisch verformbar, sodass die Spaltbreite d, d.h. der lichte Abstand zwischen dem proximalen Rohrabschnitt und dem distalen Rohrabschnitt, auf gegenüberliegenden Seiten der Rohrabschnitte in unterschiedlichem Maße und/oder in unterschiedlicher Richtung veränderbar ist. Wird der Schlauchabschnitt einseitig gestaucht oder gedehnt, verkleinert oder vergrößert sich die Spaltbreite d des ringförmigen Spalts entsprechend auf einer Seite, sodass der proximale Rohrabschnitt und der distale Rohrabschnitt relativ zueinander verschwenkt werden.

In einer Ausführungsform ist der Schlauchabschnitt beidseitig des ringförmigen Spalts an dem proximalen Rohrabschnitt und dem distalen Rohrabschnitt befestigt. Vorzugsweise ist der Schlauchabschnitt stumpf auf die Stirnseiten des proximalen und des distalen Rohrabschnittes aufgesetzt verklebt, verschweißt oder angespritzt. In einer anderen Ausführungsform weisen die einander zugewandten Enden der Rohrabschnitte einen Falz zur bündigen und glatten Aufnahme des komplementär ausgestalten Schlauchabschnittes auf. Der Schlauchabschnitt verbindet wie eine Muffe den proximalen Rohrabschnitt und den distalen Rohrabschnitt für die Durchleitung von Atemgas.

In einer Ausführungsform weist das Gelenk zusätzlich zu dem zuvor beschriebenen Schlauchabschnitt einen zweiten Schlauchabschnitt auf, der konzentrisch zu dem ersten Schlauchabschnitt angeordnet ist, wobei der erste Schlauchabschnitt radial außenliegend mit dem proximalen Rohrabschnitt und dem distalen Rohrabschnitt verbunden und der zweite Schlauchabschnitt radial innenliegend mit dem proximalen Rohrabschnitt und dem distalen Rohrabschnitt verbunden ist. Die zwei Schlauchabschnitte verbinden den proximalen Rohrabschnitt und den distalen Rohrabschnitt sowohl auf der Innenseite des Kanülenrohrs, d.h. im zentralen Lumen des Kanülenrohrs, als auch auf der Außenseite des Kanülenrohrs. Der erste Schlauchabschnitt und/oder der zweite Schlauchabschnitt überspannen den ringförmigen Spalt und sind beidseitig des ringförmigen Spalts an dem proximalen Rohrabschnitt und dem distalen Rohrabschnitt befestigt. Beispielsweise ist der eine oder sind beide Schlauchabschnitte durch Verkleben, Verschweißen oder Anspritzen an den proximalen und distalen Rohrabschnitten befestigt.

Vorzugsweise weist der eine Schlauchabschnitt des Gelenks, oder falls das Gelenk aus zwei Schlauchabschnitten besteht, beide Schlauchabschnitte, eine geringere Wandstärke als die mittlere Wandstärke des proximalen Rohrabschnitts im Anschlussbereich des Gelenks auf. Beispielsweise ist die mittlere Wandstärke des ersten Schlauchabschnittes und/oder des zweiten Schlauchabschnittes ¼ der mittleren Wandstärke des proximalen Rohrabschnitts im Anschlussbereich des Gelenks.

In einer Ausführungsform ist der Schlauchabschnitt stumpf mit den Enden der proximalen und distalen Rohrabschnitte verbunden. Somit füllt der Schlauchabschnitt den ringförmigen Spalt zwischen dem proximalen Rohrabschnitt und dem distalen Rohrabschnitt aus. Der Schlauchabschnitt kann sich nahtlos an den proximalen Rohrabschnitt und den distalen Rohrabschnitt anschließen. Der Schlauchabschnitt hat in seinem proximalen Anschlussbereich eine Wandstärke, die der Wandstärke des proximalen Rohrabschnittes entspricht, und in seinem distalen Anschlussbereich eine Wandstärke, die der Wandstärke des distalen Rohrabschnittes entspricht. In einer etwas modifizierten Ausführungsform hat der der Schlauchabschnitt eine größere Wandstärke als der proximale Rohrabschnitt und der distale Rohrabschnitt, mit je einer ringförmigen Nut in den Stirnseiten des Schlauchabschnittes für die Aufnahme der Enden des proximalen und distalen Rohrabschnitte in diesen Nuten. Die sich über den Spalt in axialer Länge des Kanülenrohrs hinaus und in den Bereich des proximalen und distalen Rohabschnittes erstreckenden Abschnitte des Schlauchabschnittes sind beidseitig des Spaltes an dem proximalen Rohrabschnitt und dem distalen Rohrabschnitt befestigt, vorzugsweise verklebt, verschweißt oder angespritzt.

Um das Gelenk zu stabilisieren, weist das Gelenk in einer Ausführungsform eine Mehrzahl von Kunststoffstegen, vorzugsweise zwei diametral gegenüberliegend angeordnete Kunststoffstege auf, die den ringförmigen Spalt überbrücken und den proximalen Rohrabschnitt mit dem distalen Rohrabschnitt verbinden. Vorzugsweise sind die Kunststoffstege gleichmäßig in Umfangsrichtung des ringförmigen Spalts verteilt angeordnet. Die Anordnung der Kunststoffstege ist geeignet, die Freiheitsgrade des Gelenks einzuschränken. Weist das Gelenk beispielsweise zwei Kunststoffstege auf, die in einem Winkelabstand von 180° oder auch etwas weniger in Umfangsrichtung des ringförmigen Spalts angeordnet sind, weist das Gelenk, ähnlich einem Scharniergelenk, eine bevorzugte Schwenkrichtung in etwa parallel zu den Kunststoffstegen auf. In einer Ausführungsform sind die Stege seitlich angeordnet, sodass mit dem Gelenk der Biegewinkel des Kanülenrohrs vergrößert oder verkleinert werden kann, zusätzlich eine Verbiegbarkeit in seitliche Richtung reduziert wird. Die Kunststoffstege können aus dem gleichen Kunststoff wie die Rohrabschnitte oder aus einem im Vergleich hierzu weicheren Kunststoff gefertigt sein. Gleichzeitig ist das Gelenk durch die Stege gegenüber axialen Kräften versteift.

In einer Ausführungsform ist die axiale Spaltbreite d des ringförmigen Spalts 1 mm bis 10 mm, vorzugsweise 3 mm bis 4 mm. Derartige Spaltbreiten verhindern, dass sich bei einer maximalen Gelenkbewegung das zentrale Lumen des Kanülenrohrs verschließt und die Atmung durch die Tracheostomiekanüle wesentlich erschwert wird.

In einer Ausführungsform wird das Gelenk aus einem Wandabschnitt des Kanülenrohres gebildet der den proximalen Rohrabschnitt vom distalen Rohrabschnitt trennt, wobei dieser Wandabschnitt eine reduzierte Wandstärke aufweist.

Hinsichtlich der Vorrichtung zur Atemluftzuführung, insbesondere Endotrachealtubus oder Tracheostomiekanüle, jeweils bestehend aus einer Innenkanüle und einer Außenkanüle mit einem Tubus, wobei der Tubus der Außenkanüle ein proximales Ende mit einer proximalen Öffnung und ein distales Ende mit einer distalen Öffnung aufweist, wird die erfindungsgemäße Aufgabe dadurch gelöst, dass die Innenkanüle eine erfindungsgemäße Tracheostomiekanüle ist, wie sie zuvor beschrieben wurde, und wobei der Tubus der Innenkanüle derart bemessen und in den Tubus der Außenkanüle einbringbar ist, dass der Ringabschnitt der Innenkanüle mit dem Cuff über das distale Ende der Außenkanüle hinaus übersteht. Konkret besteht eine solche Vorrichtung aus einer herkömmlichen Tracheostomiekanüle (Außenkanüle) in Kombination mit der erfindungsgemäßen Tracheostomiekanüle (Innenkanüle).

In einer Ausführungsform der Vorrichtung zur Atemluftzuführung weist der Tubus der Außenkanüle auf seinem äußeren Umfang einen Cuff auf. Mit dem auf dem äußeren Umfang des Tubus der Außenkanüle angeordneten Cuff ist der Tubus der Außenkanüle unabhängig von dem Cuff der Innenkanüle gegenüber der Innenwand der Trachea abdichtbar. Der Cuff der Außenkanüle ergänzt die Abdichtfunktion des Cuffs der Innenkanüle und umgekehrt. Der Cuff der Außenkanüle und der Cuff der Innenkanüle können im Wechsel betrieben werden, sodass die Trachea durch einen wechselnden Druck in den beiden Cuffs wechselseitig be- und entlastet werden kann.

Die erfindungsgemäße Kombination aus Außenkanüle und Innenkanüle zeichnet sich gegenüber dem Stand der Technik, der im Prinzip schon Kombinationen aus Außenkanüle und Innenkanüle zeigt, dadurch aus, dass sowohl die Außenkanüle als auch die Innenkanüle jeweils einen eigenen Cuff aufweist, der unabhängig von dem jeweils anderen verwendbar ist, wobei die Innenkanüle für die Verwendung ihres Cuffs entsprechend länger ist als die Außenkanüle. Bei dieser erfindungsgemäßen Kombination und für den Fall, dass die Innenkanüle nicht ausgetauscht werden muss, ist es im Übrigen nicht erforderlich, dass sie den oben erwähnten verjüngten Abschnitt aufweist, die Innenkanüle könnte dann nämlich (mit Ihrem proximalen Ende) auch in das distale Ende einer Außenkanüle eingeführt werden, ohne dass der Cuff der Innenkanüle durch die Außenkanüle hindurchgeführt werden muss. In jedem Fall ist aber der Tubus der Innenkanüle länger als der Tubus der Außenkanüle, so dass der Cuff der Innenkanüle außerhalb des distalen Endes der Außenkanüle freiliegt, während das proximale Ende der Innenkanüle aus dem proximalen Ende der Außenkanüle herausragt oder zumindest von der proximalen Seite her gut zugänglich ist. Die Innenkanüle könnte dann im Gebrauch der Kombination überwiegend inaktiv bleiben, d. h. ihr Cuff würde nicht aufgebläht werden, da der Cuff der Außenkanüle die Trachea außerhalb der Kanülen abdichten würde. Lediglich das Lumen der Innenkanüle würde dabei für die Hindurchleitung der Atemluft genutzt werden. Erst für einen Wechsel der Außenkanüle oder zur zwischenzeitlichen Entlastung der Innenwand der Trachea im Abdichtungsbereich würde der Cuff der Innenkanüle aufgebläht werden, wobei danach der Cuff der Außenkanüle dann entleert werden könnte. Dies könnte auch im gleichmäßigen Wechsel geschehen, wenn die Trachea eines Patienten einer besonderen Schonung bedarf und möglichst oft von dem lokalen Druck eines Cuffs entlastet werden soll. Der Wechsel der Außenkanüle könnte weiterhin wie oben beschreiben erfolgen. Im Übrigen könnte bei dieser Variante einer erfindungsgemäßen Kombination die Innenkanüle sämtliche bereits beschriebenen und beanspruchten Merkmale einer Innenkanüle aufweisen mit Ausnahme des verjüngten distalen Abschnittes. Der Cuff der Innenkanüle muss jeweils nur bei Bedarf aufgebläht werden, kann aber auch als doppelte Sicherung gegen eindringendes Sekret zusätzlich zu dem Cuff der Außenkanüle verwendet werden.

Die Innenkanüle und Außenkanüle der Vorrichtung zur Atemluftzuführung sind derart aufeinander abgestimmt, dass der Tubus der Innenkanüle in den Tubus der Außenkanüle einbringbar ist. In einem Zustand in dem die Innenkanüle in die Außenkanüle eingebracht ist, stehen der Ringabschnitt der Innenkanüle mit dem kleineren Außendurchmesser D_{TR} und der in diesem Abschnitt angeordneten Cuff freiliegend über das distale Ende des Tubus der Außenkanüle über. Vorzugsweise steht das proximale Ende des Tubus der Innenkanüle über das proximale Ende des Tubus der Außenkanüle hervor. Bei einer dauerhaften Kombination aus Außen- und Innenkanüle, bei welcher die Innenkanüle nicht zwischenzeitlich entfernt werden muss, ist es, wie bereits erwähnt, nicht erforderlich, dass das distale Ende der Innenkanüle einen kleineren Außendurchmesser aufweist als der Hauptabschnitt, weil dann die Innenkanüle auch von dem distalen Ende der Außenkanüle her eingeführt werden kann.

Bei einer solchen Kombination müsste man aber gegebenenfalls darauf verzichten, die Innenkanüle durch einen fest verbundenen proximalen Abschnitt größeren Durchmessers an der Außenkanüle zu sichern. Alle anderen Merkmale von Außenkanüle und Innenkanüle, wie sie vorstehend beschrieben wurden, einschließlich eines mit dem proximalen Ende der Innenkanüle lösbar verbundenen Konnektors, können aber auch bei einer von vornherein vorgesehenen Kombination aus einer in eine Außenkanüle mit einer eingesetzten Innenkanüle ohne verjüngten distalen Abschnittvorgesehen sein.

In einer Ausführungsform weist die Innenkanüle und/oder die Außenkanüle Mittel zum lösbaren aneinander Befestigen auf. Diese Mittel sind entweder so ausgestaltet, dass die Innenkanüle in Richtung ihres proximalen Endes aus einer Außenkanüle herausziehbar und/oder die Außenkanüle in Richtung ihres proximalen Endes von der Innenkanüle abziehbar ist. Die Mittel zum lösbaren Befestigen der Innenkanüle an einer Außenkanüle verhindern, dass die Innenkanüle ungewollt weiter in die Außenkanüle rein- oder aus dieser herausrutscht. Vorzugsweise umfasst das Mittel zum Befestigen Rastnasen, einen Bajonettverschluss, Gewinde und/oder eine Überwurfmutter. Die Mittel zum lösbaren Befestigen sind vorzugsweise an dem proximalen Ende des Tubus der Innenkanüle und/oder dem proximalen Ende des Tubus der Außenkanüle angeordnet.

Es versteht sich, dass die Außenkanüle der erfindungsgemäßen Vorrichtung zur Atemluftzuführung auch die Merkmale der erfindungsgemäßen Innenkanüle aufweisen kann. Insbesondere kann der Cuff der Außenkanüle genau wie der zuvor beschriebene Cuff der Innenkanüle ausgestaltet sein. Die Absaugöffnung der Außenkanüle kann analog zu der Absaugöffnung der Innenkanüle ausgestaltet sein. Die Außenkanüle kann eine Absaugleitung aufweisen, die mit der Absaugöffnung der Außenkanüle verbunden ist und im Übrigen analog zu der Absaugleitung der Innenkanüle ausgestaltet sein kann. Außerdem kann die erfindungsgemäße Innenkanüle bei entsprechender Länge und Form auch zusammen mit einem Endotrachealtubus verwendet werden, um dessen Wechsel zu erleichtern und für den Patienten sicherer zu machen.

In einer Ausführungsform weist die Innenkanüle der Vorrichtung zur Atemluftzuführung ein Gelenk auf, wobei die Innenkanüle und die Außenkanüle derart aufeinander abgestimmt sind, dass das Gelenk der Innenkanüle in einem Bereich des Tubus angeordnet ist, der aus dem distalen Ende des Tubus der Außenkanüle hervorsteht.

Weitere Vorteile, Merkmale und Anwendungsmöglichkeiten der vorliegenden Erfindung werden anhand der folgenden Beschreibung einer Ausführungsform und den dazugehörigen Figuren deutlich, wobei gleiche Bezugszeichen auf gleiche Elemente verweisen. Es zeigen:
- Figur 1:: eine Innenkanüle gemäß einer Ausführungsform der vorliegenden Erfindung,
- Figur 2:: die Innenkanüle aus der Figur 1 mit einem leeren und an den Außenumfang des Tubus der Innenkanüle angelegten Cuff, und
- Figur 3:: die Innenkanüle gemäß der Ausführungsform aus den Figuren 1 und 2 in einem Zustand in dem die Innenkanüle in den Tubus einer Außenkanüle einer erfindungsgemäßen Vorrichtung zur Atemluftzuführung eingebracht ist.

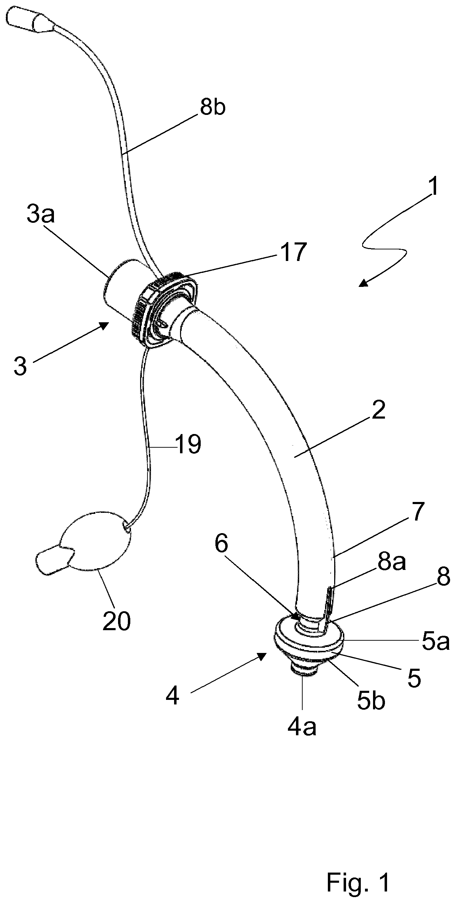

In der Figur 1 ist eine Innenkanüle 1 gemäß einer Ausführungsform der vorliegenden Erfindung in einer Perspektivansicht gezeigt. Die Innenkanüle 1 weist einen bogenförmig gekrümmten Tubus 2 mit einem proximalen Ende 3 und einem distalen Ende 4 auf. An dem proximalen Ende 3 ist eine proximale Öffnung 3a und an dem distalen Ende 4 eine distale Öffnung 4a angeordnet. Der die proximale Öffnung 3a und die distale Öffnung 4a verbindende Tubus 2 dient der Durchleitung von Atemgas. Das proximale Ende 3 ist mit einer Beatmungsvorrichtung zur Atemluftzuführung verbindbar, wobei das proximale Ende 3 hierfür einen 15 mm-Konnektor 11 aufweist. Das distale Ende 4 wird durch ein Tracheostoma in die Trachea eines Patienten eingeführt.

Nahe des distalen Endes 4 des Tubus ist an seinem äußeren Umfang ein Cuff 5 angeordnet, genauer ist der Cuff 5 in einem Ringabschnitt 6 angeordnet, der sich zumindest über die axiale Länge des Cuffs 5 erstreckt und einen kleineren Außendurchmesser D_{TR} als der Außendurchmesser D_{TPC} des Tubus in dem proximal vor dem Cuff 5 liegenden Hauptabschnitt 7 aufweist.

Der Cuff 5 ist eine Niederdruckmanschette, deren Inneres mit einem Fluid beaufschlagbar ist, sodass der Cuff 5 vollständig entfaltbar und an die Innenwand einer Trachea eines Patienten anlegbar ist. Zum Beaufschlagen mit einem Fluid ist der Cuff 5 über eine Fluidleitung 19 mit einem Kontrollballon mit Füllventil 20 verbunden. Das Fluid ist vorzugsweise Luft. In seinem vollständig gefüllten und in der Figur 1 gezeigten Zustand, hat der Cuff 5 eine maximale axiale Ausdehnung von 5 mm bis 30 mm.

Der Cuff 5 hat einen distalen Abschnitt 5b, der sich in einem Zustand, in dem der Cuff 5 vollständig befüllt ist, in Richtung des distalen Endes 4 des Tubus 2 verjüngt. Der sich radial verjüngende Abschnitt 5b verhindert oder erschwert das Blockieren der distalen Tubusöffnung durch einen weitgehend entleerten Cuff. Der Cuff 5 schließt bei der gezeigten Ausführungsform nicht direkt an das freie distale Ende 4 des Tubus 2 an, sondern hat einen axialen Abstand zwischen dem distalen Ansatzpunkt des Cuffs 5 und dem freien distalen Ende 4 des Tubus 2. Der axiale Abstand ist zwischen 1 mm und 10 mm groß und soll zusätzlich verhindern, dass sich der Cuff 5 in seinem entleerten Zustand beim Zurückziehen der Innenkanüle 1 aus dem Tubus einer Außenkanüle vor die distale Öffnung 4 des Tubus 2 der Innenkanüle 1 legt.

Der proximale Abschnitt 5a des Cuffs 5 weist eine Tellerform auf, wobei sich auf dem radial in etwa senkrecht zur Längsachse erstreckenden Abschnitt in einem Zustand, in dem die Innenkanüle 1 in eine Trachea eingeführt ist und der Cuff 5 vollständig befüllt ist, Sekret aus dem subglottischen Raum der Trachea sammeln kann. Möglicherweise angesammeltes Sekret kann über eine Absaugöffnung 8 abgesaugt werden. Die Absaugöffnung 8 ist proximal zu dem Cuff 5 innerhalb des Ringabschnittes 6 angeordnet und mit einer Absaugleitung 8a verbunden. Die Absaugleitung 8a ist in die Wand des Tubus 2 integriert und geht an dem proximalen Ende 3 des Tubus 2 in einen Schlauchfortsatz 8b über. Der Schlauchfortsatz 8b ist mit einer hier nicht dargestellten Absaugvorrichtung verbindbar.

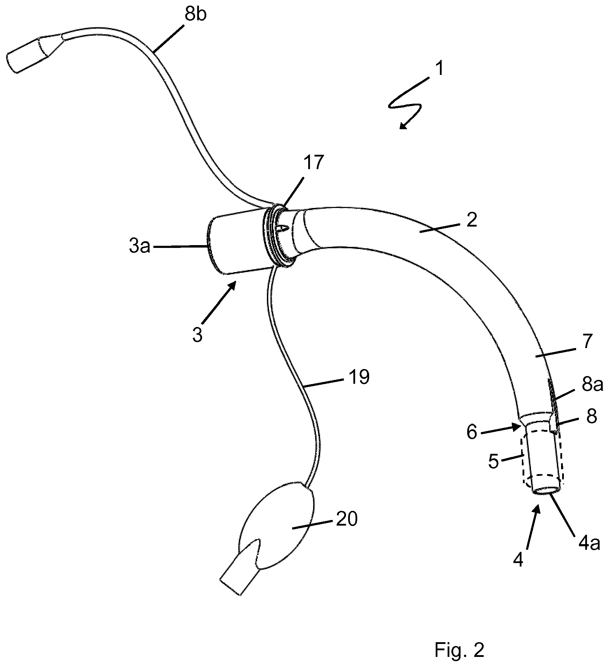

In Figur 2 ist die Innenkanüle 1 gemäß der Ausführungsform aus der Figur 1 in einem Zustand dargestellt, in dem der Cuff 5 entleert und an den Außenumfang des Ringabschnittes 6 angelegt ist. Der mit einer Strichlinie angedeutete Cuff 5 steht in diesem Zustand mit Ausnahme von dünnen und somit biegsamen Falten des Cuffs 5 nicht über den radialen Außenumfang des proximal vor dem Cuff 5 liegenden Hauptabschnitts 7 des Tubus 2 über. Auf diese Weise stört der Cuff 5 beim Einführen des Tubus 2 der Innenkanüle 1 in einen Tubus einer Außenkanüle nicht, denn die dünnen Falten des Cuffs 5 schmiegen sich beim Einführen problemlos an die Innenkanülenwand des Ringabschnittes 6 bzw. an das bereits eng anliegende Cuffmaterial an.

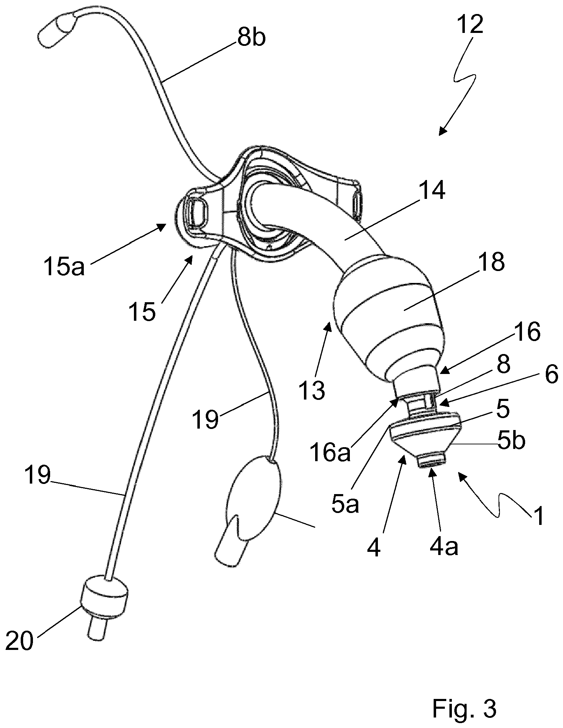

Die Figur 3 zeigt die zuvor im Zusammenhang mit den Figuren 1 und 2 beschriebene Innenkanüle 1 in einem Zustand, in dem die Innenkanüle 1 in den Tubus 14 einer Außenkanüle 13 eingeführt ist. Die insgesamt als Vorrichtung zur Atemluftzuführung 12 bezeichnete Ausführungsform besteht aus der zuvor beschriebenen Innenkanüle 1 und einer Außenkanüle 13. Die Außenkanüle 13 hat einen bogenförmig gekrümmten Tubus 14 mit einem proximalen Ende 15 und einem distalen Ende 16. Das proximale Ende 15 weist eine proximale Öffnung 15a und das distale Ende 16 eine distale Öffnung 16a auf. Der Tubus 2 der Innenkanüle 1 ist länger als der Tubus 14 der Außenkanüle 13 und derart bemessen, dass er nach dem Einbringen der Innenkanüle 1 mit seinem Ringabschnitt 6 mit dem Cuff 5 über das das distale Ende 16 der Außenkanüle 13 übersteht, wobei auch das proximale Ende der Innenkanüle 1 noch aus dem proximalen Ende der Außenkanüle 13 hervorsteht. Auch die in dem Ringabschnitt 6 angeordnete Absaugöffnung 8 liegt jenseits des distalen Endes 16 der Außenkanüle 12 frei.

Der Außendurchmesser D_{TPC} des proximal vor dem Ringabschnitt 6 angeordneten Hauptabschnittes 7 des Tubus 2 der Innenkanüle 1 ist er geringfügig kleiner als der Innendurchmesser D_{AT} des Tubus 14 der Außenkanüle 13 ist. Die Außenfläche des Abschnittes 7 liegt demnach mit geringem Spiel, vorzugsweise 0,1 mm < Durchmesserdifferenz < 0,5 mm, an der Innenfläche des Tubus 14 an.

Die Innenkanüle 1 ist lösbar an der Außenkanüle 13 befestigt. Die Innenkanüle 1 weist hierzu an dem proximalen Ende 3 des Tubus 2 Befestigungsmittel 17 auf, wie sie in der Figur 1 dargestellt sind.

Die Außenkanüle 13 weist darüber hinaus ein Kanülenschild 21 und einen Cuff 18 auf. Der Cuff 18 ist eine befüllbare Niederdruckmanschette, die an dem äußeren Umfang des Tubus 14 der Außenkanüle 13 angeordnet ist. Der Cuff 18 ist mit einer Fluidleitung 19 an ein Kontrollballon mit Füllventil 20 angeschlossen. In seinem befüllten Zustand liegt der Cuff 18 an der Innenwand der Trachea eines Patienten an und dichtet die Außenkanüle 13 gegenüber der Trachea ab.

Für Zwecke der ursprünglichen Offenbarung wird darauf hingewiesen, dass sämtliche Merkmale, wie sie sich aus der vorliegenden Beschreibung, den Zeichnungen und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen. Auf die umfassende, explizite Darstellung sämtlicher denkbarerer Merkmalskombinationen wird hier nur der Kürze und der Lesbarkeit der Beschreibung wegen verzichtet.

Während die Erfindung im Detail in den Zeichnungen und der vorangehenden Beschreibung dargestellt und beschreiben wurde, erfolgt diese Darstellung und Beschreibung lediglich beispielhaft und ist nicht als Beschränkung des Schutzbereichs gedacht, so wie er durch die Ansprüche definiert wird. Die Erfindung ist nicht auf die offenbarten Ausführungsformen beschränkt.

Abwandlungen der offenbarten Ausführungsformen sind für den Fachmann aus den Zeichnungen, der Beschreibung und den beigefügten Ansprüchen offensichtlich. In den Ansprüchen schließt das Wort "aufweisen" nicht andere Elemente oder Schritte aus, und der unbestimmte Artikel "eine" oder "ein" schließt eine Mehrzahl nicht aus. Die bloße Tatsache, dass bestimmte Merkmale in unterschiedlichen Ansprüchen beansprucht sind, schließt ihre Kombination nicht aus. Bezugszeichen in den Ansprüchen sind nicht als Beschränkung des Schutzbereichs gedacht.

### Bezugszeichenliste

- 1: Innenkanüle
- 2: Tubus
- 3: proximales Ende des Tubus 2
- 3a: proximale Öffnung
- 4: distales Ende des Tubus 2
- 4a: distale Öffnung
- 5: Cuff
- 5a: proximaler Abschnitt des Cuffs 5
- 5b: distaler Abschnitt des Cuffs 5
- 6: Ringabschnitt
- 7: Hauptabschnitt
- 8: Absaugöffnung
- 8a: Absaugleitung
- 8b: Schlauchfortsatz
- 9: stufenförmiger Übergang
- 10a: distaler Ansatzpunkt des Cuffs 5
- 10b: proximaler Ansatzpunkt des Cuffs 5
- 11: Konnektor
- 12: Vorrichtung zur Atemluftzuführung
- 13: Außenkanüle
- 14: Tubus der Außenkanüle 13
- 15: proximales Ende des Tubus 14
- 15a: proximale Öffnung
- 16: distales Ende des Tubus 14
- 16a: distale Öffnung
- 17: Befestigungsmittel
- 18: Cuff mit angedeuteten Falten
- 19: Fluidleitung
- 20: Kontrollballon mit Füllventil
- 21: Kanülenschild

## Patentansprüche

1. Tracheostomiekanüle (1) mit einem Tubus (2), welcher ein proximales Ende (3) mit einer proximalen Öffnung (3a) und ein distales Ende (4) mit einer distalen Öffnung (4a) und einen an seinem äußeren Umfang nahe des distalen Endes (4) angeordneten Cuff (5) aufweist, wobei der Tubus (2) an seinem distalen Ende (4a) einen sich zumindest über die axiale Länge des Cuffs (5) erstreckenden verjüngten Ringabschnitt (6) hat, dessen Außendurchmesser D_{TR} kleiner als der Außendurchmesser des Tubus D_{TPC} in dem proximal vor dem Ringabschnitt (6) liegenden Hauptabschnitt (7) ist, und wobei die Durchmesserdifferenz zwischen Ringabschnitt im Bereich des dort angebrachten Cuffs (5) und Hauptabschnitt so bemessen ist, dass der äußere Umfang des Cuffs (5) in einem leeren und an den äußeren Umfang des Tubus (2) angelegten Zustand nicht über den äußeren Umfang des Tubus (2) in dem proximal vordem Ringabschnitt (6) liegenden Hauptabschnitt (7) übersteht, **dadurch gekennzeichnet, dass** der Ringabschnitt (6) mit dem kleineren Außendurchmesser D_{TR} eine Absaugöffnung (8) aufweist, die proximal zu dem Cuff (5) an dem äußeren Umfang des Tubus (2) angeordnet ist.

2. Tracheostomiekanüle (1) nach Anspruch 1, **dadurch gekennzeichnet, dass** die Länge des verjüngten Ringabschnittes (6) mindestens der Länge des Cuffs entspricht und weniger als ein Drittel der Länge des Tubus beträgt und wobei das proximale Ende des Cuffs um mindestens zwei Drittel der Tubuslänge von dem proximalen Ende des Tubus beabstandet ist.

3. Tracheostomiekanüle (1) nach Anspruch 1 oder 2, **dadurch gekennzeichnet, dass** der Ringabschnitt (6) vorzugsweise im Bereich der Absaugöffnung (8) einen stufenförmigen Übergang (9) zwischen dem Außendurchmesser D_{TR} und D_{TPC} aufweist.

4. Tracheostomiekanüle (1) nach einem der vorstehenden Ansprüche, **dadurch gekennzeichnet, dass** die Absaugöffnung (8) mit einer Absaugleitung (8a) verbunden ist, wobei die Absaugleitung (8a) mit dem Tubus (2) verklebt oder in die Wand des Tubus (2) integriert ist.

5. Tracheostomiekanüle (1) nach einem der vorstehenden Ansprüche, **dadurch gekennzeichnet, dass** der Cuff (5) in seinem gefüllten Zustand eine maximale axiale Ausdehnung von 5 mm bis 30 mm, vorzugsweise 10 mm bis 20 mm, aufweist.

6. Tracheostomiekanüle (1) nach einem der vorstehenden Ansprüche, **dadurch gekennzeichnet, dass** der Cuff (5) einen proximalen Abschnitt (5a) aufweist, dessen Außendurchmesser sich in einem gefüllten Zustand des Cuffs (5) nach proximal verjüngt, und/oder der Cuff (5) einen distalen Abschnitt (5b) aufweist, dessen Außendurchmesser sich in einem gefüllten Zustand des Cufffs (5) nach distal verjüngt.

7. Tracheostomiekanüle (1) nach einem der vorstehenden Ansprüche, **dadurch gekennzeichnet, dass** der Abstand zwischen dem freien distalen Ende (4) des Tubus und dem distalen Ansatzpunkt (10a) des Cuffs (5) zwischen 1 mm und 10 mm, vorzugsweise zwischen 5 mm und 10 mm, ist.

8. Tracheostomiekanüle (1) nach einem der vorstehenden Ansprüche, **dadurch gekennzeichnet, dass** der Abstand zwischen dem freien distalen Ende (4) des Tubus und dem proximalen Ansatzpunkt (10b) des Cuffs (5) zwischen 15 mm und 40 mm ist.

9. Tracheostomiekanüle (1) nach einem der vorstehenden Ansprüche, **dadurch gekennzeichnet, dass** der äußere Umfang des Tubus (2) an seinem proximalen Ende (3) einen Außendurchmesser D_{TP} hat, der größer als der Außendurchmesser des Tubus D_{TPC} proximal zu dem Ringabschnitt (6) ist, wobei vorzugsweise der Außendurchmesser D_{TP} um mindestens 5 % und maximal 20 %, vorzugsweise um etwa 10 %, größer ist als der Außendurchmesser D_{TPC}.

10. Tracheostomiekanüle (1) nach einem der vorstehenden Ansprüche, **dadurch gekennzeichnet, dass** das proximale Ende (3) des Tubus (2) kein Kanülenschild aufweist und/oder das proximale Ende (3) des Tubus (2) einen 15 mm Konnektor (11) aufweist.

11. Tracheostomiekanüle (1) nach einem der vorstehenden Ansprüche, **dadurch gekennzeichnet, dass** die Tracheostomiekanüle (1) ein Gelenk aufweist, welches das proximale Ende (3) und das distale Ende (4) des Tubus (2) miteinander verbindet und mindestens eine begrenzte Beweglichkeit insbesondere Verschwenkbarkeit der Achse des distalen Endes (4) bezüglich der Achse des proximalen Endes (3) ermöglicht.

12. Vorrichtung zur Atemluftzuführung (12), insbesondere Endotrachealtubus oder Tracheostomiekanüle, bestehend aus einer Innenkanüle (1) sowie einer Außenkanüle (13) mit einem Tubus (14), wobei der Tubus (14) der Außenkanüle (13) ein proximales Ende (15) mit einer proximalen Öffnung (15a) und ein distales Ende (16) mit einer distalen Öffnung (16a) aufweist, **dadurch gekennzeichnet, dass** die Innenkanüle (1) eine Tracheostomiekanüle (1) nach einem der Ansprüche 1 bis 11 ist, wobei der Tubus (2) der Innenkanüle (1) derart bemessen und in den Tubus (14) der Außenkanüle (13) einbringbar ist, dass der Ringabschnitt (6) der Innenkanüle (1) mit dem Cuff (5) über das distale Ende (16) der Außenkanüle (13) übersteht.

13. Vorrichtung zur Atemluftzuführung (12) nach Anspruch 13, **dadurch gekennzeichnet, dass** die Innenkanüle (1) und/oder die Außenkanüle (13) Mittel (17) zum lösbaren aneinander Befestigen aufweisen, und/oder der Tubus (14) der Außenkanüle (13) auf seinem äußeren Umfang einen Cuff (18) aufweist.

14. Vorrichtung zur Atemluftzuführung (12) nach einem der Ansprüche 13 oder 14, **dadurch gekennzeichnet, dass** der Tubus (14) der Außenkanüle (13) einen Innendurchmesser D_{AT} aufweist, der mit dem Außendurchmesser des Tubus (2) D_{TPC} der Innenkanüle (1) proximal zu dem Ringabschnitt (6) anschließt.

## Claims

1. A tracheostomy cannula (1) comprising a tube (2) which has a proximal end (3) with a proximal opening (3a) and a distal end (4) with a distal opening (4a) and a cuff (5) arranged at its outer periphery near the distal end (4), wherein at its distal end (4) the tube (2) has a tapered ring portion (6) which extends at least over the axial length of the cuff (5) and whose outside diameter D_{TR} is less than the outside diameter of the tube D_{TPC} in the main portion (7) proximally in front of the ring portion (6), and wherein the difference in diameter between the ring portion in the region of the cuff (5) disposed there and the main portion is such that in an empty state applied to the outer periphery of the tube (2) the outer periphery of the cuff (5) does not project beyond the outer periphery of the tube (2) in the main portion (7) which is proximally in front of the ring portion (6), **characterised in that** the ring portion (6) with the smaller outside diameter D_{TR} has a suction opening (8) which is arranged proximally relative to the cuff (5) at the outer periphery of the tube (2).

2. A tracheostomy cannula (1) according to claim 1 **characterised in that** the length of the tapered ring portion (6) corresponds at least to the length of the cuff and is less than a third of the length of the tube and wherein the proximal end of the cuff is spaced from the proximal end of the tube by at least two thirds of the tube length.

3. A tracheostomy cannula (1) according to claim 1 or claim 2 **characterised in that** the ring portion (6) has a step-shaped transition (9) between the outside diameter D_{TR} and D_{TPC}, preferably in the region of the suction opening (8).

4. A tracheostomy cannula (1) according to one of the preceding claims **characterised in that** the suction opening (8) is connected to a suction line (8a), wherein the suction line (8a) is glued to the tube (2) or is integrated into the wall of the tube (2).

5. A tracheostomy cannula (1) according to one of the preceding claims **characterised in that** in its filled state the cuff (5) has a maximum axial extent of 5 mm to 30 mm, preferably 10 mm to 20 mm.

6. A tracheostomy cannula (1) according to one of the preceding claims **characterised in that** the cuff (5) has a proximal portion (5a), the outside diameter of which tapers in the proximal direction in a filled state of the cuff (5) and/or the cuff (5) has a distal portion (5b), the outside diameter of which tapers in the distal direction in a filled state of the cuff (5).

7. A tracheostomy cannula (1) according to one of the preceding claims **characterised in that** the spacing between the free distal end (4) of the tube and the distal attachment point (10a) of the cuff is between 1 mm and 10 mm, preferably between 5 mm and 10 mm.

8. A tracheostomy cannula (1) according to one of the preceding claims **characterised in that** the spacing between the free distal end (4) of the tube and the proximal attachment point (10b) of the cuff is between 15 mm and 40 mm.

9. A tracheostomy cannula (1) according to one of the preceding claims **characterised in that** the outer periphery of the tube (2) at its proximal end (3) is of an outside diameter D_{TP} which is larger than the outside diameter of the tube D_{TPC} proximally relative to the ring portion (6), wherein preferably the outside diameter D_{TP} is larger than the outside diameter D_{TPC} by at least 5% and a maximum of 20%, preferably by about 10%.

10. A tracheostomy cannula (1) according to one of the preceding claims **characterised in that** the proximal end (3) of the tube (2) does not have a cannula shield and/or the proximal end (3) of the tube (2) has a 15 mm connector (11).

11. A tracheostomy cannula (1) according to one of the preceding claims **characterised in that** the tracheostomy cannula (1) has a joint which connects the proximal end (3) and the distal end (4) of the tube (2) together and permits at least limited mobility, in particular pivotability, of the axis of the distal end (4) relative to the axis of the proximal end (3).

12. Apparatus (12) for supplying respiratory air, in particular an endotracheal tube or tracheostomy cannula, comprising an inner cannula (1) and an outer cannula (13) having a tube (14), wherein the tube (14) of the outer cannula (13) has a proximal end (15) with a proximal opening (15a) and a distal end (16) with a distal opening (16a), **characterised in that** the inner cannula (1) is a tracheostomy cannula (1) according to one of claims 1 to 11, wherein the tube (2) of the inner cannula (1) is of such a size and can be introduced into the tube (14) of the outer cannula (13) in such a way that the ring portion (6) of the inner cannula (1) with the cuff (5) projects beyond the distal end (16) of the outer cannula (13).

13. Apparatus (12) for supplying respiratory air according to claim 12 **characterised in that** the inner cannula (1) and/or the outer cannula (13) have means (17) for releasably fixing them together and/or the tube (14) of the outer cannula (13) has a cuff (18) on its outer periphery.

14. Apparatus (12) for supplying respiratory air according to one of claims 12 and 13 **characterised in that** the tube (14) of the outer cannula (13) is of an inside diameter D_{AT} which adjoins with the outside diameter of the tube (2) D_{TPC} of the inner cannula (1) proximally relative to the ring portion (6).

## Revendications

1. Canule de trachéotomie (1) comportant un tube (2) qui présente une extrémité proximale (3) dotée d'une ouverture proximale (3a) et une extrémité distale (4) dotée d'une ouverture distale (4a) ainsi qu'un ballonnet (5) agencé sur sa circonférence extérieure à proximité de l'extrémité distale (4), le tube (2) présentant à son extrémité distale (4a) une section annulaire effilée (6) s'étendant au moins sur la longueur axiale du ballonnet (5), dont le diamètre extérieur D_{TR} est inférieur au diamètre extérieur du tube D_{TPC} dans la section principale (7) située du côté proximal avant la section annulaire (6), et la différence de diamètre entre la section annulaire dans la zone du ballonnet (5) qui y est attachée et la section principale étant telle que la circonférence extérieure du ballonnet (5) dans un état vide et appliqué à la circonférence extérieure du tube (2) ne dépasse pas de la circonférence extérieure du tube (2) dans la section principale (7) située du côté proximal avant la section annulaire (6), **caractérisée en ce que** la section annulaire (6) avec le diamètre extérieur D_{TR} plus petit présente une ouverture d'aspiration (8) qui est agencée du côté proximal par rapport au ballonnet (5) à la circonférence extérieure du tube (2).

2. Canule de trachéotomie (1) selon la revendication 1, **caractérisée en ce que** la longueur de la section annulaire effilée (6) correspond au moins à la longueur du ballonnet et représente moins d'un tiers de la longueur du tube et l'extrémité proximale du ballonnet étant espacée de l'extrémité proximale du tube d'au moins deux tiers de la longueur de tube.

3. Canule de trachéotomie (1) selon la revendication 1 ou 2, **caractérisée en ce que** la section annulaire (6) présente de préférence dans la zone de l'ouverture d'aspiration (8) une transition en gradins (9) entre les diamètres extérieurs D_{TR} et D_{TPC}.

4. Canule de trachéotomie (1) selon l'une quelconque des revendications précédentes, **caractérisée en ce que** l'ouverture d'aspiration (8) est reliée à une conduite d'aspiration (8a), la conduite d'aspiration (8a) étant collée au tube (2) ou étant intégrée dans la paroi du tube (2).

5. Canule de trachéotomie (1) selon l'une quelconque des revendications précédentes, **caractérisée en ce que** le ballonnet (5), dans son état rempli, présente une extension axiale maximum de 5 mm à 30 mm, de préférence de 10 mm à 20 mm.

6. Canule de trachéotomie (1) selon l'une des revendications précédentes, **caractérisée en ce que** le ballonnet (5) présente une section proximale (5a) dont le diamètre extérieur rétrécit vers le côté proximal lorsque le ballonnet (5) est rempli, et/ou le ballonnet (5) présente une section distale (5b) dont le diamètre extérieur rétrécit vers le côté distal lorsque le ballonnet (5) est rempli.

7. Canule de trachéotomie (1) selon l'une quelconque des revendications précédentes, **caractérisée en ce que** la distance entre l'extrémité distale libre (4) du tube et le point de départ distal (10a) du ballonnet (5) est comprise entre 1 mm et 10 mm, de préférence entre 5 mm et 10 mm.

8. Canule de trachéotomie (1) selon l'une quelconque des revendications précédentes, **caractérisée en ce que** la distance entre l'extrémité distale libre (4) du tube et le point de départ proximal (10b) du ballonnet (5) est comprise entre 15 mm et 40 mm.

9. Canule de trachéotomie (1) selon l'une quelconque des revendications précédentes, **caractérisée en ce que** la circonférence extérieure du tube (2) présente à son extrémité proximale (3) un diamètre extérieur D_{TP} qui est supérieur au diamètre extérieur du tube D_{TPC} proximal à la section annulaire (6), le diamètre extérieur D_{TP} étant de préférence au moins 5 % et au maximum 20 %, de préférence d'environ 10 %, supérieur au diamètre extérieur D_{TPC}.

10. Canule de trachéotomie (1) selon l'une quelconque des revendications précédentes, **caractérisée en ce que** l'extrémité proximale (3) du tube (2) ne présente pas de protection de canule et/ou l'extrémité proximale (3) du tube (2) présente un connecteur (11) de 15 mm.

11. Canule de trachéotomie (1) selon l'une quelconque des revendications précédentes, **caractérisée en ce que** la canule de trachéotomie (1) présente une articulation qui relie l'extrémité proximale (3) et l'extrémité distale (4) du tube (2) l'une à l'autre et permet au moins une mobilité limitée, en particulier une possibilité de pivotement de l'axe de l'extrémité distale (4) par rapport à l'axe de l'extrémité proximale (3).

12. Dispositif d'alimentation en air respirable (12), en particulier tube endotrachéal ou canule de trachéotomie, constitué d'une canule interne (1) et d'une canule externe (13) comportant un tube (14), le tube (14) de la canule externe (13) présentant une extrémité proximale (15) dotée d'une ouverture proximale (15a) et une extrémité distale (16) dotée d'une ouverture distale (16a), **caractérisé en ce que** la canule interne (1) est une canule de trachéotomie (1) selon l'une des revendications 1 à 11, le tube (2) de la canule interne (1) étant dimensionné et conçu pour être inséré dans le tube (14) de la canule externe (13) de manière telle que la section annulaire (6) de la canule interne (1) avec le ballonnet (5) dépasse de l'extrémité distale (16) de la canule externe (13).

13. Dispositif d'alimentation en air respirable (12) selon la revendication 12, **caractérisé en ce que** la canule interne (1) et/ou la canule externe (13) présente(nt) des moyens (17) de fixation amovible de l'une à l'autre, et/ou le tube (14) de la canule externe (13) présente un ballonnet (18) sur sa circonférence extérieure.

14. Dispositif d'alimentation en air respirable (12) selon l'une quelconque des revendications 13 ou 14, **caractérisé en ce que** le tube (14) de la canule externe (13) présente un diamètre intérieur D_{AT} qui constitue la suite du diamètre extérieur du tube (2) D_{TPC} de la canule interne (1) du côté proximal de la section annulaire (6).
